# EUROPEAN PATENT SPECIFICATION

(11) **EP 4 343 326 B1**
(45) Date of publication and mention of the grant of the patent: **12.03.2025**
(21) Application number: 22196631.0
(22) Date of filing: 20.09.2022
(51) Int. Cl.: G01N 33/543, G01N 33/58, G01N 33/536

(54) **BIOLOGICAL CONJUGATES HAVING AN PHOTOGENERATED ACIDIC OR BASIC RELEASABLE DETECTION MOIETY ON TOP OF BIOLOGICAL TISSUES**
BIOLOGISCHE KONJUGATE MIT EINER FOTOGENERIERTEN SAUREN ODER BASISCHEN FREISETZBAREN DETEKTIONSEINHEIT AUF BIOLOGISCHEM GEWEBE
CONJUGUÉS BIOLOGIQUES AYANT UNE FRACTION DE DÉTECTION PHOTOGÉNÉRÉE, ACIDE OU BASIQUE, LIBÉRABLE SUR LA PARTIE SUPÉRIEURE DE TISSUS BIOLOGIQUES

(43) Date of publication of application: 27.03.2024
(73) Proprietor: Miltenyi Biotec B.V. & Co. KG, 51429 Bergisch Gladbach (DE)
(72) Inventor: PERBOST, Michel, 51429 Bergisch Gladbach (DE); SOLIMAN, Sameh, 51429 Bergisch Gladbach (DE); GUAN, Xiao-Pei, 51429 Bergisch Gladbach (DE); MARMA, Mong, 51429 Bergisch Gladbach (DE)
(74) Representative: Kisters, Michael Marcus

(56) References cited:
- WO-A1-2020/201450
- WO-A1-2020/207963
- US-A- 5 338 641
- US-A1- 2017 275 669
- YAGNIK GARGEY ET AL: "Highly Multiplexed Immunohistochemical MALDI-MS Imaging of Biomarkers in Tissues", JOURNAL OF THE AMERICAN SOCIETY FOR MASS SPECTROMETRY, vol. 32, no. 4, 2021, pages 977 - 988, XP055924565

## Description

### BACKGROUND

The present invention is directed to a process for detection or identification of target moieties or target cells or from a cell sample by labelling the target moieties or target cells with a conjugate having a detection moiety and an antigen or an oligonucleotide recognizing moiety linked via an acidic or basic degradable spacer, wherein the acid, or base, used for degrading the spacer is provided in form of a spatially photoactivable precursor.

After detecting the target moieties, the precursor is activated or photocleaved by radiation and the acid, or base, for degrading the spacer is released, thereby removing at least the detection moiety from the labelled target moiety.

### BACKGROUND

It is long known to detect cells by using conjugates comprising a fluorescent moiety and antigen recognizing moiety. Furthermore, conjugates have been developed from which the fluorescent moiety can be removed, thereby allowing re-staining of the cells which in turn allows detecting multiple target moieties.

It is also known that fluorescently labelled oligonucleotides can be used to detect a target sequence and the fluorescent moiety can be cleaved off later to perform subsequent multiple set of hybridization and detection to increase the power of decoding or detection. Gargey Yagnik et al: Journal of the American Society for Mass Spectrometry (2021) vol. 32, no. 4, pages 977-988 discloses the highly multiplexed immunohistochemical MALDI-MS imaging of biomarkers in tissues.

### SUMMARY

Surprisingly it was found that conjugates composed of an antigen binding moiety linked via an acidic or basic degradable spacer to a detection moiety can be degraded by activating or photocleaving a precursor of an acid or a base. After release, the biological specimen can be subjected to the same or a different conjugate again without interference of the previous label.

Object of the invention is therefore a method for detecting a target moiety in a sample of biological specimens by
a) providing at least one conjugate with the general formula (I) Xₙ - P - Yₘ, with X is a detection moiety, P an acidic or basic degradable spacer and Y an antigen or oligonucleotide recognizing moiety and n, m are integers between 1 and 100 and wherein X and Y are covalently bound to P,
b) binding the conjugate to the target moiety via the antigen or oligonucleotide recognizing moiety Y, thereby labelling the target moiety,
c) detecting the target moiety labelled with the conjugate by detecting the detecting moiety X,
d) providing at least one precursor which is capable of releasing an acid or base when provided with radiation wherein the acid or base is capable of cleaving P,
e) activating the precursor by providing radiation, thereby releasing the acid or base capable of cleaving P, and
f) degrading spacer P with the released acid or base, thereby cleaving the detection moiety X from the conjugated detection moiety.

Activation of the precursor may be achieved by photocleaving the precursor into at least two subuntits wherein at least one of the subunits is an acid and/or a base.

Preferable, by acidic or basic degrading spacer P, the antigen or oligonucleotide recognizing moiety Y is cleaved from the detection moiety.

### BRIEF DESCRIPTION OF THE DRAWINGS

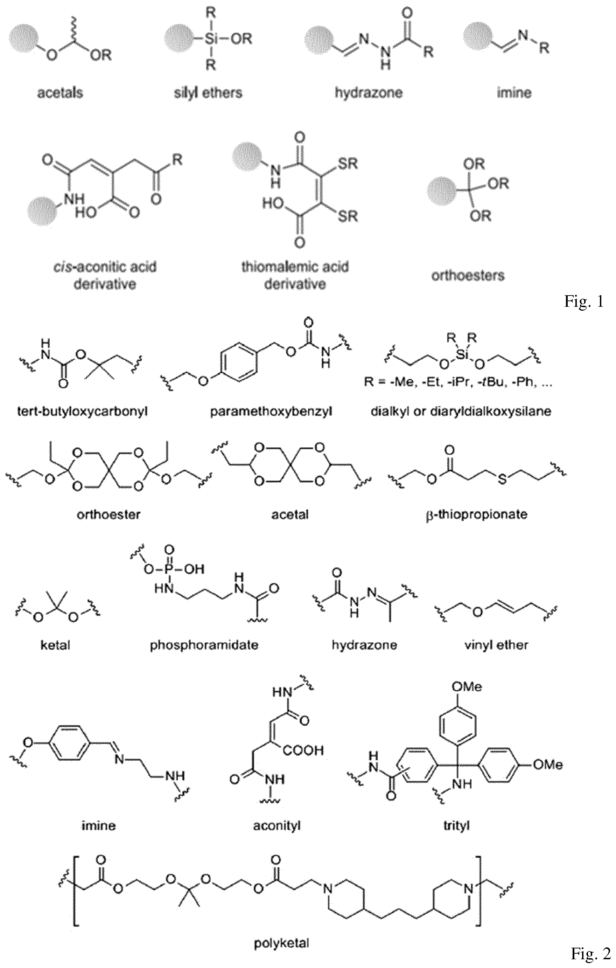

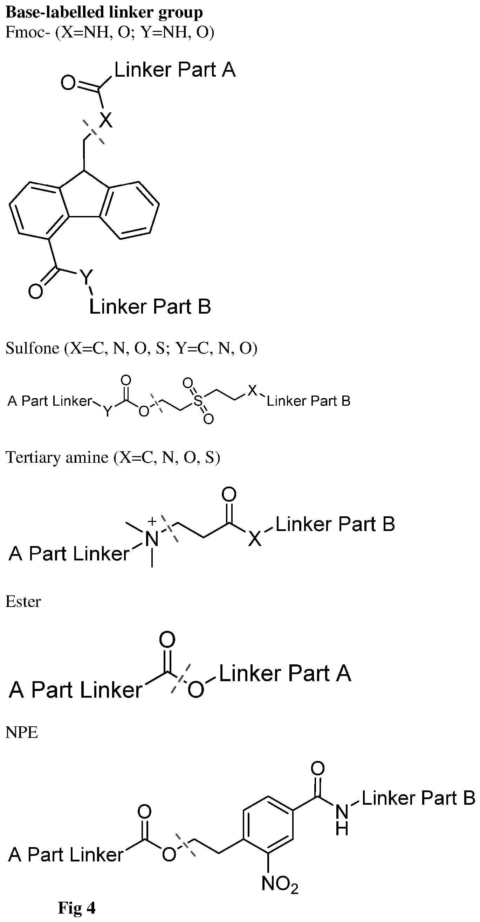

Fig. 1 to 4 show examples of units for acidic or basic degradable spacer P. Fig. 2 is taken from G. Lerche et al, Bioorg Chem. 20 (2021) 571-582)

### DETAILED DESCRIPTION

The term "cleaving the detection moiety X from the conjugate" means that the bond between X and P is abrogated and detection moiety X may be removed from the target for example by dissociation or washing.

Optionally, the antigen recognizing moiety Y is also be removed from the target by abrogating the bond between X and P.

The process of the invention may be performed in one or more sequences of the steps a) to g). After each sequence, the detection moiety and optionally the antigen recognizing moiety is released (removed) from the target moiety. Especially when the biological specimens are living cells which shall be further processed or analysed, the method of the invention has the advantage of providing unlabelled cells.

After and/or before each step a) - g), a washing step can be performed to remove unwanted material like unbound conjugates or released detection moieties from the sample.

### Target moiety

The target moiety to be detected with the method of the invention can be on any biological specimen, like tissues slices, cell aggregates, suspension cells, or adherent cells. The cells may be living or dead. Preferable, target moieties are antigens expressed intracellular or extracellular on biological specimen like whole animals, organs, tissues slices, cell aggregates, or single cells of invertebrates, (e.g., Caenorhabditis elegans, Drosophila melanogaster), vertebrates (e.g., *Danio rerio* (Hamilton, 1822) *, Xenopus laevis,* (Daudin 1802)) and mammalians (e.g., *Mus musculus,* Linnaeus, 1758, *Homo sapiens,* Linneaeus, 1758)).

### Detection moiety

The detection moiety X of the conjugate may be any moiety possessing a property or function which can be used for detection purposes like those selected from the group consisting of chromophore moiety, fluorescent moiety, phosphorescent moiety, luminescent moiety, light absorbing moiety, radioactive moiety, and transition metal isotope mass tag moiety.

Suitable fluorescent moieties are those known from the art of immunofluorescence technologies, e.g., flow cytometry or fluorescence microscopy. In these embodiments of the invention, the target moiety labeled with the conjugate is detected by exciting the detection moiety X and detecting the resulting emission (photoluminescence). In this embodiment, the detection moiety X is preferable a fluorescent moiety.

Useful fluorescent moieties might be protein-based, such as phycobiliproteins, polymeric, such as polyfluorenes, small organic molecule dyes, such as xanthenes, like fluorescein, or rhodamines, cyanines, oxazines, coumarins, acridines, oxadiazoles, pyrenes, pyrromethenes, or metallo-organic complexes, such as Ru, Eu, Pt complexes. Besides single molecule entities, clusters of fluorescent proteins or small organic molecule dyes, as well as nanoparticles, such as quantum dots, upconverting nanoparticles, gold nanoparticles, dyed polymer nanoparticles can also be used as fluorescent moieties.

Another group of photoluminescent detection moieties are phosphorescent moieties with time-delayed emission of light after excitation. Phosphorescent moieties include metallo-organic complexes, such as Pd, Pt, Tb, Eu complexes, or nanoparticles with incorporated phosphorescent pigments such as lanthanide doped SrAl₂O₄.

In another embodiment of the invention the target labeled with the conjugate is detected without prior excitation by irradiation. In this embodiment the detection moiety can be a radioactive label. They may be in the form of radioisotope labeling by exchanging nonradioactive isotopes for their radioactive counterparts, such as tritium, ³²P, ³⁵S or ¹⁴C, or introducing covalently bound labels, such as ¹²⁵I, which is bound to tyrosine, ¹⁸F within fluorodeoxyglucose, or metallo-organic complexes, i.e. ⁹⁹Tc-DTPA.

In another embodiment the detection moiety is capable of causing chemiluminescence, i.e. horseradish peroxidase label in the presence of luminol.

In another embodiment of the invention the target labeled with the conjugate is not detected by radiation emission, but by absorption of UV, visible light, or NIR radiation. Suitable light-absorbing detection moieties are light absorbing dyes without fluorescence emission, such as small organic molecule quencher dyes like N-aryl rhodamines, azo dyes, and stilbenes.

In another embodiment, the light-absorbing detection moieties X can be irradiated by pulsed laser light, generating an photoacoustic signal.

In another embodiment of the invention the target labeled with the conjugate is detected by mass spectrometric detection of a transition metal isotope. Transition metal isotope mass tag labels might be introduced as covalently bound metallo-organic complexes or nanoparticle component. Known in the art are isotope tags of lanthanides and adjacent late transition elements.

The detection moiety X can be covalently or non-covalently coupled to the spacer P. Methods for covalently or non-covalently conjugation are known by persons skilled in the art. In case of a covalent bound between the detection moiety X and the spacer P, a direct reaction of an activated group either on the detection moiety or on the spacer P with a functional group on either the spacer P or on the detection moiety X or via an heterobifunctional linker molecule, which is firstly reacted with one and secondly reacted with the other binding partner is possible.

For example, a large number of heterobifunctional compounds are available for linking to entities. Illustrative entities include: azidobenzoyl hydrazide, N-[4-(p-azidosalicylamino)butyl]-3'-[2'-pyridyldithio]propionamide), bis-sulfosuccinimidyl suberate, dimethyladipimidate, disuccinimidyltartrate, N-y-maleimidobutyryloxysuccinimide ester, N-hydroxy sulfosuccinimidyl-4- azidobenzoate, N-succinimidyl [4-azidophenyl]-1,3'-dithiopropionate, N-succinimidyl [4-iodoacetyl]aminobenzoate, glutaraldehyde, succinimidyl-[(N-maleimidopropionamido) polyethyleneglycol] esters (NHS-PEG-MAL), and succinimidyl 4-[N-maleimidomethyl]cyclohexane-1-carboxylate. A preferred linking group is 3-(2-pyridyldithio)propionic acid N-hydroxysuccinimide ester (SPDP), or 4-(N-maleimidomethyl)-cyclohexane-1-carboxylic acid N-hydroxysuccinimide ester (SMCC) with a reactive sulfhydryl group on the detection moiety and a reactive amino group on the spacer P.

A quasi-covalent binding of the detection moiety X to the spacer P can be achieved with binding systems providing a dissociation constant of ≤ 10⁻⁹ M, e.g., Biotin-Avidin binding interaction.

### Acidic or basic degradable spacer P

Suitable acidic or basic degradable spacers P may comprise acidic or basic cleavable functional units selected from the group consisting of acetals, silyl esters, hydrazine, imine, esters, phosphoramidite, hydrazine, vinyl ether, trityl, aconitril, oxycarbonyl, paramethoxybenzyl, ketal, polyketal, orthoesters, thiomalemic acid, in a linker with alkyl, polyethyleneglycol, polysaccharides, proteins, peptides, depsipeptides, polyesters, nucleic acids segments and derivatives thereof.

The radiation used for activating or photocleaving the precursor depends on the chemical nature of the precursor, but is usually in the range of 200 to 800 nm.

### Precursor

Suitable precursors comprises functional units selected from the group consisting of diazonium salts, perhalomethyltriazines, Halobisphenyl, o-nitrobenzaldehyde, sulfonates, imidylsulfonyl esters, diaryliodonium salts, sulfonium salts, diazosulfonate, diarylsulfones, 1,2-diazoketones, 2-diazo 1-oxo 5 sulfonyl, 2-diazo 1-oxo 4 sulfonyl, diazomethyl ketone, diazoMeldrum's acid, arylazidederivatives, benzocarbonates, carbamates, dimethoxybenzoiinyl carbonates, dimethoxybenzoiinyl carbamates, o-nitrobenzyloxy carbonates, o-nitrobenzyloxy carbamates, nitrobenzene sulpenyl, o-nitroanilines, nitrobenzylcarbamate, 3',5'-dimethoxybenzoin carbamates.

### Antigen recognizing moiety Y

The term "antigen recognizing moiety Y" refers to any kind of antibody, fragmented antibody or fragmented antibody derivatives, directed against the target moieties expressed on the biological specimens, like antigens expressed intracellular or extracellular on cells. The term relates to fully intact antibodies, fragmented antibody or fragmented antibody derivatives, e.g., Fab, Fab', F(ab')2, sdAb, scFv, di-scFv, nanobodies. Such fragmented antibody derivatives may be synthesized by recombinant procedures including covalent and non-covalent conjugates containing these kind of molecules. Further examples of antigen recognizing moieties are peptide/MHC-complexes targeting TCR molecules, cell adhesion receptor molecules, receptors for costimulatory molecules, artificial engineered binding molecules, e.g., peptides or aptamers which target, e.g., cell surface molecules.

The "antigen recognizing moiety Y" may also comprise oligonucleotide which recognize appropriate target moieties of the biological specimens, like other nucleotides. Preferable, the oligonucleotides comprise 10-200 nucleotides.

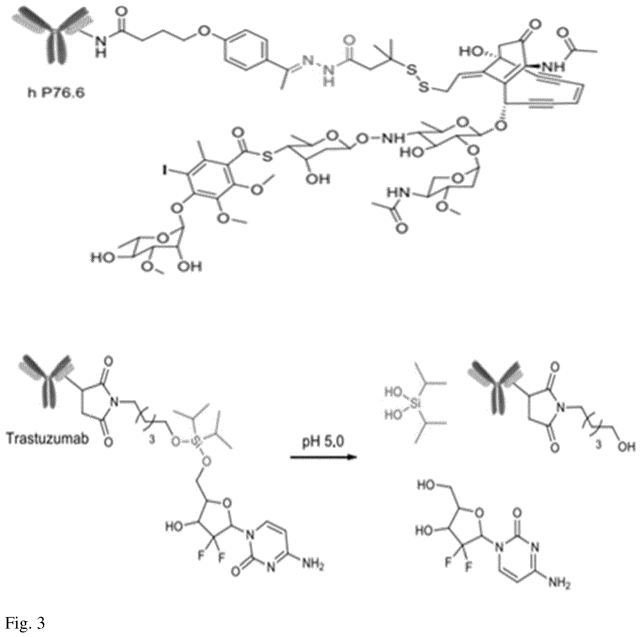

The conjugate used in the method of the invention may comprise up to 100, preferable 1- 20 antigen recognizing moieties Y. The interaction of the antigen recognizing moiety with the target antigen can be of high or low affinity. Binding interactions of a single low-affinity antigen recognizing moiety is too low to provide a stable bond with the antigen. Low-affinity antigen recognizing moieties can be multimerized by conjugation to the enzymatically degradable spacer P to furnish high avidity. When the spacer P is enzymatically cleaved in step g), the low-affinity antigen recognizing moieties will be monomerized which results in a complete removal of the detection moiety X, the spacer P and the antigen recognizing moiety Y (Fig. 3 b). High-affinity antigen recognizing moieties provide a stable bond which results in a removal of the detection moiety X and the spacer P during step g).

Preferable, the term "Antigen recognizing moiety Y" refers to an antibody directed against antigen expressed by the biological specimens (target cells) intracellular, like IL2, FoxP3, CD154, or extracellular, like CD3, CD14, CD4, CD8, CD25, CD34, CD56, and CD133.

The antigen recognizing moieties Y, especially antibodies, can be coupled to the spacer P through side chain amino or sufhydryl groups. In some cases, the glycosidic side chain of the antibody can be oxidized by periodate resulting in aldehyde functional groups.

The antigen recognizing moiety Y can be covalently or non-covalently coupled to the spacer P. Methods for covalent or non-covalent conjugation are known by persons skilled in the art and the same as mentioned for conjugation of the detection moiety X.

The method of the invention is especially useful for detection and/or isolation of specific cell types from complex mixtures and may comprise more than one sequential or parallel sequences of the steps a) - g). The method may use a variety of combinations of conjugates. For example, a conjugate may comprise antibodies specific for two different epitopes, like two different anti-CD34 antibodies. Different antigens may be addressed with different conjugates comprising different antibodies, for example, anti-CD4 and anti-CD8 for differentiation between two distinct T-cell-populations or anti-CD4 and anti-CD25 for determination of different cell subpopulations like regulatory T-cells.

The amount of acid needs to be sufficient to degrade substantially the spacer in the desired period of time. Usually, the detection signal is at least about 80%, more usually at least about 95%, preferably at least about 99% reduced. The conditions for release may be empirically optimized in terms of temperature, pH, presence of metal cofactors, reducing agents, etc. The degradation will usually be completed in at least about 15 minutes, more usually at least about 10 minutes, and will usually not be longer than about 30 minutes.

### Embodiments of the method

First, more than one precursor may be provided, wherein the different precursors are activated or photocleaved by radiation of different wavelengths.

Second, the conjugate is provided to a surface of a biological specimen and the radiation for activating the precursor is applied to spatial defined regions of the biological specimen. Obviously, the degrading of spacer P is restricted to the spatial defined regions of the biological specimen only.

Third, the sample of biological specimens has a first pH and after activating the precursor by providing radiation, at least a part of the sample of biological specimens has a second pH; wherein the difference of the first and second pH is at least +-0.5, preferably at least +-1,0, more preferred at least +-2.0. However, pH range shall not exceed 4 to 10.

Forth, the radiation for activating the precursor is applied to at least one spatial defined region of the biological specimen, wherein before providing radiation, the at least one spatial defined region has a first pH and after activating the precursor by providing radiation, the at least one spatial defined region has a second pH; wherein the difference of the first and second pH is at least +-0,5, preferably at least +-1,0, more preferred at least +-2.0. However, pH range shall not exceed 4 to 10.

### Cell Detection Methods

The method and equipment to detect the target labeled with the conjugate Xₙ - P - Yₘ is determined by the detection moiety X.

In one variant of the invention the detection moiety X is a fluorescent moiety. Targets labeled with fluorochrome-conjugate are detected by exciting the fluorescent moiety X and analysing the resulting fluorescence signal. The wavelength of the excitation is usually selected according to the absorption maximum of the fluorescent moiety X and provided by LASER or LED sources as known in the art. If several different detection moieties X are used for multiple colour/parameter detection, care should be taken to select fluorescent moieties having not overlapping absorption spectra, at least not overlapping absorption maxima. In case of a fluorescent moieties as detection moiety the targets may be detected, e.g., under a fluorescence microscope, in a flow cytometer, a spectrofluorometer, or a fluorescence scanner. Light emitted by chemiluminescence can be detected by similar instrumentation omitting the excitation.

Radioactive detection moieties are detected though the radiation emitted by the radioactive isotopes. Suitable instrumentation for detection of radioactive radiation include, for example, scintillation counters. In case of beta emission electron microscopy can also be used for detection.

Transition metal isotope mass tag moieties are detected by mass spectrometric methods such as ICP-MS, which is integrated in mass cytometry instrumentation.

### Use of the method

The method of the invention can be used for various applications in research, diagnostics and cell therapy.

In a first variant of the invention, biological specimens like cells are detected for counting purposes i.e. to establish the amount of cells from a sample having a certain set of antigens recognized by the antigen recognizing moieties of the conjugate.

In a second variant, one or more populations of biological specimens are detected from the sample and separated as target cells. This variant may be used for purification of target cells, for example, in clinical research, diagnostics, and immunotherapy. In this variant, one or more sorting steps may be performed after any of the steps a) to g) and the optionally washing steps.

Suitable for such separations are especially flow sorters, e.g., FACS or MEMS-based cell sorter systems, for example as disclosed in EP14187215.0 or EP14187214.3.

In another variant of the invention, the location of the target moieties like antigens on the biological specimens recognized by the antigen recognizing moieties of the conjugate is determined. Such techniques are known as "Multi Epitope Ligand Cartography", "Chip-based Cytometry" or "Multioymx" and are described, for example, in EP 0810428, EP1181525, EP 1136822 or EP1224472. In this technology, cells are immobilized and contacted with antibodies coupled to fluorescent moiety. The antibodies are recognized by the respective antigens on the biological specimen (for example on a cell surface) and after removing the unbound marker and exciting the fluorescent moieties, the location of the antigen is detected by the fluorescence emission of the fluorescent moieties. In certain variants, instead of antibodies coupled to fluorescent moieties, antibodies coupled to moieties detectable for MALDI-Imaging or CyTOF can be used. The person skilled in the art is aware how to modify the technique based on fluorescent moiety to work with these detection moieties.

The location of the target moieties is achieved by a digital imaging device with a sufficient resolution und sensitivity in for the wavelength of the fluorescence radiation. The digital imaging device may be used with or without optical enlargement for example with a fluorescence microscope. The resulting images are stored on an appropriate storing device like a hard drive, for example in RAW, TIF, JPEG, or HDF5 format.

In order to detect different antigens, different antibody-conjugates having the same or different fluorescent moiety or antigen recognizing moiety Y can be provided. Since the parallel detection of fluorescence emission with different wavelengths is limited, the antibody-fluorochrome-conjugates are utilized sequentially individually or in small groups (2-10) after the other.

In yet another variant of the method according to the invention, the biological specimens - especially suspension cells - of the sample are immobilized by trapping in microcavities or by adherence.

In general, the method of the invention can be performed in several variants. For example, the conjugate not recognized by a target moiety can be removed by washing for example with buffer before the target moiety labelled with the conjugate is detected.

In a variant of the invention, at least two conjugates are provided simultaneously or in subsequent staining sequences, wherein each antigen recognizing moiety Y recognizes different antigens. In an alternative variant, at least two conjugates can be provided to the sample simultaneously or in subsequent staining sequences, wherein each conjugate comprises a different degradable spacer P which is cleaved by different enzymes. In both cases, the labelled target moieties can be detected simultaneously or sequentially. Sequential detection may involve simultaneous degrading of the spacer molecules P or subsequent degrading of the spacer molecules P with optionally intermediate removing (washing) of the non-bonded moieties.

## Claims

1. Method for detecting a target moiety in a sample of biological specimens by:
a) providing at least one conjugate with the general formula (I) Xₙ - P - Yₘ, with X is a detection moiety, P an acidic or basic degradable spacer and Y an antigen or oligonucleotide recognizing moiety and n, m are integers between 1 and 100 and wherein X and Y are covalently bound to P,
b) binding the conjugate to the target moiety via the antigen or oligonucleotide recognizing moiety Y, thereby labelling the target moiety,
c) detecting the target moiety labelled with the conjugate by detecting the detecting moiety X,
d) providing at least one precursor which is capable of releasing an acid or base when provided with radiation wherein the acid or base is capable of cleaving P,
e) activating the precursor by providing radiation, thereby releasing the acid or base capable of cleaving P, and
f) degrading spacer P with the released acid or base, thereby cleaving the detection moiety X from the conjugated detection moiety.

2. Method according to claim 1 **characterized in that** the precursor is activated by photocleaving into at least two subuntits, wherein at least one of the subunits is an acid and/or a base.

3. Method according to claims 1 or 2 **characterized by** acidic or basic degrading spacer P, the antigen or oligonucleotide recognizing moiety Y is cleaved from the detection moiety.

4. Method according to any of the claims 1 to 3 **characterized in that** the detection moiety is selected from the group consisting of chromophore moiety, fluorescent moiety, phosphorescent moiety, luminescent moiety, light absorbing moiety, radioactive moiety, transition metal and isotope mass tag moiety.

5. Method according to any of the claims 1 to 4, **characterized in that** the acidic or basic degradable spacer P comprises acidic or basic cleavable functional units selected from the group consisting of acetals, silyl esters, hydrazine, imine, esters, phosphoramidite, hydrazine, vinyl ether, trityl, aconityl, oxycarbonyl, paramethoxybenzyl, ketal, polyketal, orthoesters, thiomalemic acid, in a linker with alkyl, polyethyleneglycol, polysaccharides, proteins, peptides, depsipeptides, polyesters, nucleic acids segments and derivatives thereof.

6. Method according to any of the claims 1 to 5, **characterized in that** the antigen recognizing moiety Y is an antibody, a fragmented antibody, an fragmented antibody derivative, peptide/MHC-complexes targeting TCR molecules, cell adhesion receptor molecules, receptors for costimulatory molecules or artificial engineered binding molecules.

7. Method according to any of the claims 1 to 6, **characterized in that** the precursor comprises functional units selected from the group consisting of diazonium salts, perhalomethyltriazines, Halobisphenyl, o-nitrobenzaldehyde, sulfonates, imidylsulfonyl esters, diaryliodonium salts, sulfonium salts, diazosulfonate, diarylsulfones, 1,2-diazoketones, 2-diazo 1-oxo 5 sulfonyl, 2-diazo 1-oxo 4 sulfonyl, diazomethyl ketone, diazoMeldrum's acid, arylazidederivatives, benzocarbonates, carbamates, dimethoxybenzoiinyl carbonates, dimethoxybenzoiinyl carbamates, o-nitrobenzyloxy carbonates, o-nitrobenzyloxy carbamates, nitrobenzene sulpenyl, o-nitroanilines, nitrobenzylcarbamate, 3',5'-dimethoxybenzoin carbamates.

8. Method according to any of the claims 1 to 7, **characterized in that** more than one precursor is provided, wherein the different precursors are activated by radiation of different wavelengths.

9. Method according to any of the claims 1 to 8 **characterized in that** the sample of biological specimens has a first pH and after activating the precursor by providing radiation, at least a part of the sample of biological specimens has a second pH; wherein the difference of the first and second pH is at least +-0,5.

10. Method according to any of the claims 1 to 9, **characterized in that** the conjugate is provided to a surface of a biological specimen and the radiation for activating the precursor is applied to spatial defined regions of the biological specimen.

11. Method according to claim 10, **characterized in that** the radiation for activating the precursor is applied to at least one spatial defined region of the biological specimen, wherein before providing radiation, the at least one spatial defined region has a first pH and after activating the precursor by providing radiation, the at least one spatial defined region has a second pH; wherein the difference of the first and second pH is at least +-0,5.

## Patentansprüche

1. Verfahren zum Nachweisen einer Zielkomponente in einer Probe von biologischen Präparaten durch:
a) Bereitstellen mindestens eines Konjugats mit der allgemeinen Formel (I) Xₙ - P - Yₘ, wobei X eine Nachweiskomponente ist, P ein saurer oder basischer abbaubarer Spacer und Y eine Antigen- oder Oligonukleotid-Erkennungskomponente ist, und n, m ganze Zahlen zwischen 1 und 100 sind und wobei X und Y kovalent an P gebunden sind,
b) Binden des Konjugats an die Zielkomponente über die Antigen- oder Oligonukleotid-Erkennungskomponente Y, wodurch die Zielkomponente markiert wird,
c) Nachweisen der Zielkomponente, die mit dem Konjugat markiert ist, durch Nachweisen der Nachweiskomponente X,
d) Bereitstellen mindestens eines Vorläufers, der in der Lage ist, eine Säure oder Base freizusetzen, wenn er bestrahlt wird, wobei die Säure oder Base in der Lage ist, P zu spalten,
e) Aktivieren des Vorläufers durch Bestrahlen, wodurch die Säure oder Base, die in der Lage ist, P zu spalten, freigesetzt wird; und
f) Abbauen des Spacers P mit der freigesetzten Säure oder Base, wodurch die Nachweiskomponente X von der konjugierten Nachweiskomponente abgespalten wird.

2. Verfahren nach Anspruch 1, **dadurch gekennzeichnet, dass** der Vorläufer durch Fotospaltung in mindestens zwei Untereinheiten aktiviert wird, wobei mindestens eine der Untereinheiten eine Säure und/oder eine Base ist.

3. Verfahren nach Anspruch 1 oder 2, **gekennzeichnet durch** das saure oder basische Abbauen des Spacer P, wobei die Antigen- oder Oligonukleotid-Erkennungskomponente Y von der Nachweiskomponente abgespalten wird.

4. Verfahren nach einem der Ansprüche 1 bis 3, **dadurch gekennzeichnet, dass** die Nachweiskomponente ausgewählt ist aus der Gruppe bestehend aus einer chromophoren Komponente, einer fluoreszierenden Komponente, einer phosphoreszierenden Komponente, einer lumineszierenden Komponente, einer lichtabsorbierenden Komponente, einer radioaktiven Komponente, einem Übergangsmetall und einer Isotopenmassenmarkierungskomponente.

5. Verfahren nach einem der Ansprüche 1 bis 4, **dadurch gekennzeichnet, dass** der sauer oder basisch abbaubare Spacer P sauer oder basisch spaltbare funktionelle Einheiten umfasst, ausgewählt aus der Gruppe bestehend aus Acetalen, Silylestern, Hydrazin, Imin, Estern, Phosphoramidit, Hydrazin, Vinylether, Trityl, Aconityl, Oxycarbonyl, Paramethoxybenzyl, Ketal, Polyketal, Orthoestern, Thiomaleminsäure, in einem Linker mit Alkyl, Polyethylenglykol, Polysacchariden, Proteinen, Peptiden, Depsipeptiden, Polyestern, Nukleinsäuresegmenten und Derivaten davon.

6. Verfahren nach einem der Ansprüche 1 bis 5, **dadurch gekennzeichnet, dass** die Antigen-Erkennungskomponente Y ein Antikörper, ein fragmentierter Antikörper, ein fragmentiertes Antikörperderivat, Peptid/MHC-Komplexe, die auf TCR-Moleküle gerichtet sind, Zelladhäsionsrezeptormoleküle, Rezeptoren für kostimulatorische Moleküle oder künstlich hergestellte Bindungsmoleküle ist.

7. Verfahren nach einem der Ansprüche 1 bis 6, **dadurch gekennzeichnet, dass** der Vorläufer funktionelle Einheiten umfasst, ausgewählt aus der Gruppe bestehend aus Diazoniumsalzen, Perhalogenmethyltriazinen, Halobisphenyl, o-Nitrobenzaldehyd, Sulfonaten, Imidylsulfonylestern, Diaryliodoniumsalzen, Sulfoniumsalzen, Diazosulfonat, Diarylsulfonen, 1,2-Diazoketonen, 2-Diazo-1-oxo-5-sulfonyl, 2-Diazo-1-oxo-4-sulfonyl, Diazomethylketon, Diazo-Meldrumsäure, Arylazid-Derivaten, Benzocarbonaten, Carbamaten, Dimethoxybenzoiinylcarbonaten, Dimethoxybenzoiinylcarbamaten, o-Nitrobenzyloxycarbonaten, o-Nitrobenzyloxycarbamaten, Nitrobenzolsulphenyl, o-Nitroanilinen, Nitrobenzylcarbamat, 3',5'-Dimethoxybenzoincarbamaten.

8. Verfahren nach einem der Ansprüche 1 bis 7, **dadurch gekennzeichnet, dass** mehr als ein Vorläufer bereitgestellt wird, wobei die verschiedenen Vorläufer durch Bestrahlung mit unterschiedlichen Wellenlängen aktiviert werden.

9. Verfahren nach einem der Ansprüche 1 bis 8, **dadurch gekennzeichnet, dass** die Probe der biologischen Präparate einen ersten pH-Wert aufweist und nach Aktivierung des Vorläufers durch Bestrahlen mindestens ein Teil der Probe der biologischen Präparate einen zweiten pH-Wert aufweist, wobei die Differenz zwischen dem ersten und dem zweiten pH-Wert mindestens +-0,5 beträgt.

10. Verfahren nach einem der Ansprüche 1 bis 9, **dadurch gekennzeichnet, dass** das Konjugat auf eine Oberfläche eines biologischen Präparats bereitgestellt wird und die Bestrahlung zur Aktivierung des Vorläufers auf räumlich definierte Regionen des biologischen Präparats angewendet wird.

11. Verfahren nach Anspruch 10, **dadurch gekennzeichnet, dass** die Bestrahlung zur Aktivierung des Vorläufers auf mindestens eine räumlich definierte Region des biologischen Präparats angewendet wird, wobei vor der Bestrahlung die mindestens eine räumlich definierte Region einen ersten pH-Wert aufweist und nach der Aktivierung des Vorläufers durch Bestrahlung die mindestens eine räumlich definierte Region einen zweiten pH-Wert aufweist; wobei die Differenz zwischen dem ersten und dem zweiten pH-Wert mindestens +-0,5 beträgt.

## Revendications

1. Procédé de détection d'une fraction cible dans un échantillon de spécimens biologiques par :
a) la fourniture d'au moins un conjugué avec la formule générale (I) Xₙ-P-Yₘ, avec X qui est une fraction de détection, P un espaceur dégradable acide ou basique et Y un antigène ou une fraction reconnaissant un oligonucléotide et n, m sont des nombres entiers entre 1 et 100 et X et Y étant liés de manière covalente à P,
b) la liaison du conjugué à la fraction cible via l'antigène ou la fraction reconnaissant l'oligonucléotide Y, marquant ainsi la fraction cible,
c) la détection de la fraction cible marquée avec le conjugué en détectant la fraction de détection X,
d) la fourniture d'au moins un précurseur qui est capable de libérer un acide ou une base quand il est fourni avec un rayonnement l'acide ou la base étant capable de cliver P,
e) l'activation du précurseur en fournissant le rayonnement, libérant ainsi l'acide ou la base capable de cliver P et
f) la dégradation de l'espaceur P avec l'acide ou la base libérée, clivant ainsi la fraction de détection X de la fraction de détection conjuguée.

2. Procédé selon la revendication 1, **caractérisé en ce que** le précurseur est activé par photoclivage en au moins deux sous-unités, au moins une des sous-unités étant un acide et/ou une base.

3. Procédé selon les revendications 1 ou 2, **caractérisé par** la dégradation acide ou basique de l'espaceur P, l'antigène ou la fraction reconnaissant l'oligonucléotide Y est clivé de la fraction de détection.

4. Procédé selon l'une quelconque des revendications 1 à 3, **caractérisé en ce que** la fraction de détection est choisie dans le groupe constitué par une fraction de chromophore, une fraction fluorescente, une fraction phosphorescente, une fraction luminescente, une fraction absorbant la lumière, une fraction radioactive, un métal de transition et une fraction d'étiquette de masse d'isotope.

5. Procédé selon l'une quelconque des revendications 1 à 4, **caractérisé en ce que** l'espaceur P dégradable acide ou basique comprend des unités fonctionnelles clivables acides ou basiques choisies dans le groupe constitué par les acétals, les esters de silyle, l'hydrazine, une imine, les esters, le phosphoramidite, l'hydrazine, l'éther de vinyle, le trityle, l'aconityle, l'oxycarbonyle, le paraméthoxybenzyle, un cétal, un polycétal, les orthoesters, l'acide thiomalémique, dans un lieur avec un alkyle, le polyéthylèneglycol, les polysaccharides, les protéines, les peptides, les depsipeptides, les polyesters, les segments d'acide nucléique et leurs dérivés.

6. Procédé selon l'une quelconque des revendications 1 à 5, **caractérisé en ce que** la fraction reconnaissant l'antigène Y est un anticorps, un anticorps fragmenté, un dérivé d'anticorps fragmenté, des molécules TCR ciblant des complexes peptide/MHC, des molécules de récepteur d'adhésion cellulaire, des récepteurs pour molécules costimulatrices ou des molécules de liaison modifiées artificielles.

7. Procédé selon l'une quelconque des revendications 1 à 6, **caractérisé en ce que** le précurseur comprend des unités fonctionnelles choisies dans le groupe constitué par les sels de diazonium, les perhalogénométhyltriazines, l'halobisphényle, le o-nitrobenzaldéhyde, les sulfonates, les esters d'imidylsulfonyle, les sels de diaryliodonium, les sels de sulfonium, le diazosulfonate, les diarylsulfones, les 1,2-diazocétones, le 2-diazo 1-oxo 5 sulfonyle, le 2-diazo 1-oxo 4 sulfonyle, la diazométhyl cétone, l'acide de diazomeldrum, les dérivés d'arylazide, les benzocarbonates, les carbamates, les carbonates de diméthoxybenzoiinyle, les carbamates de diméthoxybenzoiinyle, les o-nitrobenzyloxy carbonates, les o-nitrobenzyloxy carbamates, le nitrobenzène sulpényle, les o-nitroanilines, le nitrobenzylcarbamate, les 3',5'-diméthoxybenzoïne carbamates.

8. Procédé selon l'une quelconque des revendications 1 à 7, **caractérisé en ce que** plus d'un précurseur est fourni, les différents précurseurs étant activés par rayonnement de différentes longueurs d'onde.

9. Procédé selon l'une quelconque des revendications 1 à 8, **caractérisé en ce que** l'échantillon de spécimens biologiques a un premier pH et après l'activation du précurseur en fournissant un rayonnement, au moins une partie de l'échantillon de spécimens biologiques a un second pH ; la différence du premier et du second pH étant d'au moins +-0,5.

10. Procédé selon l'une quelconque des revendications 1 à 9, **caractérisé en ce que** le conjugué est fourni à une surface d'un spécimen biologique et le rayonnement pour activer le précurseur est appliqué à des régions définies spatiales du spécimen biologique.

11. Procédé selon la revendication 10, **caractérisé en ce que** le rayonnement pour activer le précurseur est appliqué à au moins une région définie spatiale du spécimen biologique, avant de fournir le rayonnement, l'au moins une région définie spatiale ayant un premier pH et après activation du précurseur en fournissant le rayonnement, l'au moins une région définie spatiale ayant un second pH ; la différence du premier et du second pH étant d'au moins +-0,5.
